# FASCICULE DE BREVET EUROPEEN

(11) **EP 1 643 856 B1**
(45) Date de publication et mention de la délivrance du brevet: **25.04.2007**
(21) Numéro de dépôt: 04767540.0
(22) Date de dépôt: 01.07.2004
(51) Int. Cl.: A23L 1/03, A23L 1/30, A23L 1/105, A23L 1/20, A23C 9/123, C12N 1/20

(54) **PROCEDE DE PREPARATION D'UN MILIEU FERMENTE A BASE DE FIBRES VEGETALES ET SON UTILISATION POUR LA FABRICATION DE PRODUITS ALIMENTAIRES RICHES EN FIBRES**
VERFAHREN ZUR HERSTELLUNG EINES FERMENTIERTEN PRODUKTS AUF PFLANZENBALLASTSTOFFBASIS UND SEINE VERWENDUNG FÜR DIE HERSTELLUNG VON NAHRUNGSMITTELPRODUKTEN MIT ERHÖHTEM BALLASTSTOFFGEHALT
METHOD FOR PREPARING A FERMENTED MEDIUM BASED ON VEGETABLE FIBERS AND USE THEREOF FOR MAKING FIBER-ENRICHED FOOD PRODUCTS

(30) Priorité: 02.07.2003 FR 0308031; 15.07.2003 FR 0308598; 30.06.2004 FR 0407196
(43) Date de publication de la demande: 12.04.2006
(73) Titulaire: COMPAGNIE GERVAIS DANONE, 92302 Levallois-Perret (FR)
(72) Inventeur: DOUGE, Marion, F-91190 GIF SUR YVETTE (FR); DEBRU, François, F-78000 Versailles (FR); TEISSIER, Philippe, F-91300 Massy (FR)
(74) Mandataire: Goulard, Sophie
(86) Numéro de dépôt international: PCT/FR2004/001697
(87) Numéro de publication internationale: WO 2005/011403

(56) Documents cités:
- EP-A- 1 001 033
- EP-A- 1 195 095
- EP-A- 1 208 752
- FR-A- 2 771 600
- FR-A- 2 815 830
- US-A1- 2003 031 756

## Description

La présente Invention est relative à un procédé de préparation d'un milieu liquide fermenté à base de fibres végétales, au milieu liquide fermenté susceptible d'être obtenu en mettant en oeuvre ce procédé, à son utilisation pour la fabrication de produits alimentaires crus ou cuits, au procédé de fabrication de tels produits alimentaires utilisant le milieu fermenté en tant qu'ingrédient, et aux produits alimentaires ainsi obtenus.

Les fibres végétales sont constituées de polysaccharides, à l'exception de la lignine (fibres du bois). Lorsqu'elles sont ingérées par l'homme, ces fibres ne sont majoritairement pas assimilées par l'organisme car elles sont résistantes à la digestion dans l'intestin grêle, du fait de l'absence dans cette portion du tube digestif de l'équipement enzymatique nécessaire à leur dégradation. La digestion des fibres ne se fait donc que dans le côlon, sous l'action des bactéries présentes localement. Elles sont couramment utilisées dans les produits alimentaires afin de tirer partie de leurs propriétés avantageuses d'un point de vue nutritionnel.

En effet, toutes les fibres végétales ont en commun la propriété d'augmenter la sensation de satiété par l'augmentation de la viscosité et du volume du bol alimentaire. Cependant, n'étant pas ou peu assimilées, elles n'apportent que très peu de calories supplémentaires et sont donc souvent appelées « fibres diététiques » pour cette raison.

D'une manière générale, les fibres peuvent incorporer des quantités importantes d'eau et possèdent des propriétés nutritionnelles différentes selon leur solubilité. En effet, en contact avec l'eau, les fibres solubles telles que par exemple les pectines et les gommes, forment un gel visqueux et épais qui ralentit le processus de la digestion, alors que les fibres insolubles, telles que la cellulose, certaines hémicelluloses et la lignine notamment, restent en suspension et gonflent en absorbant de l'eau et ce jusqu'à vingt fois leur poids. En augmentant le volume et l'hydratation des selles, elles facilitent ainsi le transit intestinal.

La consommation quotidienne habituelle de fibres alimentaires dans les pays industrialisés est généralement de l'ordre de 15 à 20 grammes par jour et par individu, alors qu'une consommation de l'ordre de 30 à 40 grammes de fibres par jour et par personne sont recommandés. Ainsi, la pauvreté en fibres de l'alimentation a été reliée à une incidence croissante, dans les pays industrialisés, de certaines pathologies digestives telles que la constipation, la diverticulose du côlon, le cancer du côlon, les hémorroïdes, etc. La sous-consommation de fibres végétales a également été incriminée comme facteur de risque de l'obésité, notamment par le retard de l'impression de satiété qu'elle entraîne (Morais *et al.,* J. Pediatr. Gastroenterol. Nutri., 1999, **29**(2), 132-135 ; Howarth *et al.,* Nutr. Rev., 2001, **59**(5), 129-139 et Bingham *et al.,* Lancet, 2003, **361**(9368), 1496-1501).

Pour palier cette carence en fibres avérée dans l'alimentation des consommateurs des pays industrialisés, une des solutions consiste par exemple à en introduire en quantité dans les produits alimentaires, et en particulier par l'intermédiaire de céréales connues pour leur richesse en fibres.

C'est ainsi qu'il existe sur le marché différents types de produits alimentaires enrichis en fibres végétales se présentant majoritairement sous la forme de produits céréaliers cuits (crackers, substituts du pain, pains moelleux, barres énergétiques, biscuits, pâtisseries, viennoiseries), ou crus liquides ou semi-liquides tels que par exemple les produits laitiers comme les yaourts aux céréales, les boissons enrichies en fibres végétales telles que les jus de fruits ou bien encore les fourrages pour gaufrettes ou biscuits.

La grande majorité des produits céréaliers cuits est actuellement préparée à partir de farine de blé blanche. Celle-ci, par sa richesse en amidon, constitue une source d'énergie importante d'un point de vue nutritionnel. Cependant, cette farine est relativement pauvre en composés tels que fibres, protéines, vitamines et minéraux, et par conséquent, peu représentative du potentiel nutritionnel du grain de blé entier.

En revanche, l'enveloppe externe des grains de céréales et notamment celle du blé (son de blé) peut contenir jusqu'à 50 % de fibres végétales. En effet, la quantité en différentes fibres varie considérablement selon l'espèce de plante considérée et d'une partie à l'autre de celle-ci. Ainsi, contrairement aux fruits et légumes qui les renferment en petites quantités, les fibres insolubles sont prédominantes dans les céréales. A titre d'exemple, le son de blé contient principalement des polysaccharides (47 % environ) comme la cellulose et l'hémicellulose, qui sont des fibres insolubles dans l'eau et il est également composé de protéines (15 % environ), d'amidon (8 % environ) et de minéraux (6 % environ).

Ainsi, l'utilisation du son de blé, qui possède une composition plus riche en différents nutriments que d'autres céréales, en tant que source de fibres, permettrait d'améliorer la valeur nutritionnelle des produits céréaliers fabriqués.

Cependant, le son de blé ainsi que la plupart des céréales ou substrats riches en fibres présentent, au-delà de leur profil nutritionnel particulièrement intéressant, plusieurs caractéristiques qui les rendent difficilement exploitables sous leur forme brute. Tout d'abord, leur forte teneur en fibres leur confère un caractère hygroscopique qui rend leur utilisation dans un processus de fabrication et leur incorporation en quantité importante dans une pâte très difficiles. En effet, l'incorporation d'une grande quantité de fibres dans une pâte entraîne des problèmes de consistance (déchirement de la pâte, non dispersion des fibres avec les autres ingrédients...) et des contraintes de laminage lors de la fabrication des produits cuits.

De plus, de tels substrats riches en fibres sont généralement peu satisfaisants d'un point de vue organoleptique (sensation de râpeux ; goût vert, amer, astringent propre aux végétaux).

Enfin, ils sont généralement riches en composés « anti-nutritionnels », en particulier en acide phytique (3 % dans le son de blé), qui en se combinant aux cations bivalents et trivalents (calcium, magnésium, fer, zinc, molybdène par exemple), forme des complexes peu solubles, qui sont peu digérés et peu absorbés par l'organisme et qui limitent ainsi l'accessibilité de ces minéraux. Ces complexes sont donc responsables de la faible biodisponibilité des minéraux dans des produits riches en acide phytique comme le blé ou le soja. Ce phénomène est d'ailleurs à l'origine de nombreuses déficiences minérales observées dans les pays en voie de développement, où l'alimentation est essentiellement composée de céréales.

Par conséquent, les seuils d'incorporation de fibres végétales dans les produits alimentaires actuellement disponibles sur le marché restent faibles et sont généralement compris entre 3 % environ pour des pains moelleux (type pains de mie ou pains au lait), des biscuits, des crackers, des pâtisseries et 10 % environ dans le pain.

Quelques exemples de taux d'incorporation de fibres dans différents produits alimentaires sont notamment résumés dans le Tableau I ci-après :

**TABLEAU I**

| Type de produits | Taux de fibres (% par rapport au produit fini) |
|---|---|
| Cracker | 3,8 |
| Biscuit | 2,6 |
| Pain moelleux | 2,8 |
| Viennoiserie de type pain au lait | 3,9 |
| Yaourt nature aux céréales | 0,5 |
| Jus d'orange | 0,3 |

Une transformation préalable des fibres végétales est donc indispensable pour pouvoir les utiliser en quantités plus importantes dans les produits alimentaires et en exploiter ainsi le potentiel nutritionnel.

Parmi les différentes techniques de transformation préalable des fibres végétales déjà connues, on peut en premier lieu citer la micronisation. Si cette technique permet d'augmenter légèrement les quantités de fibres pouvant être incorporées dans les produits alimentaires cuits (permettant en particulier d'atteindre des taux d'incorporation des fibres de l'ordre de 5 à 7 % environ pour les crackers), cette technique entraîne par ailleurs une perte du caractère abrasif des fibres ainsi que de leurs propriétés nutritionnelles. Par ailleurs, la micronisation des fibres ne permet pas de supprimer de façon significative les composés « anti-nutritionnels » tels que l'acide phytique, ni d'améliorer les propriétés organoleptiques des produits alimentaires les contenant.

La fermentation des fibres par des micro-organismes (bactéries, champignons, moisissures, levures, etc) est également souvent reconnue pour sa contribution à l'amélioration des qualités nutritionnelles du substrat. De plus, la fermentation contribue à l'amélioration des caractéristiques organoleptiques des substrats riches en fibres, et permet ainsi une meilleure acceptabilité des produits alimentaires enrichis en fibres par le consommateur.

Parmi les micro-organismes qui sont habituellement utilisés pour fermenter les substrats riches en fibres, on peut notamment citer les bactéries lactiques qui sont des bactéries à Gram positif, non sporulantes, microaérophiles et dont le principal produit de fermentation est l'acide lactique.

Ainsi, Salmenkallio-Marttila M. *et al.* (Cereal Chem., 2001, **78**(4), 429-435) ont notamment comparé l'effet de la fermentation spontanée de fractions de son de blé avec celui d'une fermentation par ajout d'une levure seule ou en mélange avec une bactérie lactique du genre *Lactobacillus brevis* sur la qualité d'un pain enrichi en son de blé. Ces Auteurs ont ainsi montré que la préfermentation du son de blé par addition d'une levure associée ou non avec la bactérie lactique *Lactobacillus brevis* améliore le volume du pain, la structure de la mie et la durée de conservation du pain enrichi en son de blé et que de tels effets bénéfiques ne sont pas observés par incorporation de son de blé ayant subi une fermentation spontanée.

Cependant, après incorporation de 20 % de son de blé, le pain obtenu renferme seulement 10 % de fibres totales, ce qui correspond aux seuils d'incorporation moyens décrits dans l'art antérieur.

Par ailleurs, la bactérie lactique *Lactobacillus brevis* utilisée dans cette étude appartient à la catégorie des bactéries dites hétérofermentaires, c'est-à-dire produisant du gaz carbonique lors de l'assimilation des sucres du substrat, contrairement aux bactéries dites homofermentaires. Cette caractéristique est un inconvénient rédhibitoire à l'utilisation de bactéries hétérofermentaires dans certains secteurs alimentaires comme la fabrication de produits crus du type boissons ou produits frais (yaourts, par exemple) dans lesquels tout dégagement de gaz carbonique est à éviter de par la nature même de ces produits.

Il n'existe donc actuellement aucun procédé permettant d'améliorer de façon significative les quantités de fibres pouvant être incorporées dans des produits alimentaires crus ou cuits.

C'est donc afin de remédier à l'ensemble de ces problèmes majeurs que les Inventeurs ont mis au point ce qui fait l'objet de l'Invention.

Ils se sont notamment donnés pour but de pourvoir à un procédé de préparation d'un milieu liquide fermenté à base de fibres végétales, lequel milieu pouvant être ensuite utilisé à titre d'ingrédient lors de la fabrication de produits alimentaires crus ou cuits afin d'incorporer des quantités significativement plus importantes de fibres végétales totales dans les produits finis (augmentation d'un facteur allant de 2 à 10) que celles habituellement incorporées dans les produits alimentaires actuellement disponibles sur le marché et de participer ainsi à l'augmentation de leur concentration dans la prise alimentaire.

De plus, les Inventeurs se sont également donnés pour but de pourvoir à un procédé de préparation d'un milieu liquide fermenté riche en fibres végétales permettant d'utiliser directement le milieu fermenté issu de la mise en oeuvre d'un tel procédé en tant qu'ingrédient lors de la fabrication de produits alimentaires crus ou cuits, sans qu'il soit nécessaire de modifier l'appareillage des lignes industrielles classiques en dehors de l'étape de préfermentation.

La présente Invention a donc pour premier objet un procédé de préparation d'un milieu liquide fermenté à base de fibres végétales solubles et insolubles, caractérisé par le fait qu'il comprend au moins une étape d'incubation d'un milieu liquide comprenant de l'eau et au moins un mélange de fibres végétales, avec au moins une bactérie lactique homofermentaire choisie parmi les *Lactobacillus plantarum, Lactobacillus paraplantarum* et *Lactobacillus pentosus* ; ledit mélange de fibres végétales renfermant au moins 40 % en poids de fibres insolubles par rapport au poids total de fibres ; le substrat végétal contenant les fibres n'étant ni préhydrolysé ou prédigéré enzymatiquement ni gélatinisé et sa teneur en fibres totales étant d'au moins 20 % en poids par rapport au poids total de sa matière sèche.

Les Inventeurs ont en effet mis en évidence que le procédé conforme à l'Invention présente non seulement l'avantage de permettre l'incorporation de quantités importantes de fibres dans des produits d'alimentation crus ou cuits, mais qu'il permet également d'obtenir un ingrédient à valeur nutritionnelle ajoutée notamment par la modification du rapport fibres solubles/fibres insolubles, l'élimination de facteurs anti-nutritionnels, l'augmentation de la solubilité des minéraux. Ainsi, par ce procédé, il est en particulier possible d'augmenter les quantités pondérales de fibres totales habituellement contenues dans les produits alimentaires d'un facteur allant de 2 à 10.

Selon le procédé conforme à l'Invention, le milieu liquide dans lequel est réalisée l'étape d'incubation comprend de préférence d'environ 55 à 90 % en poids d'eau et d'environ 10 à 45 % en poids de fibres végétales.

Le mélange de fibres végétales est avantageusement un mélange de fibres de céréales et/ou de légumineuses choisies parmi les fibres extraites du blé, de l'orge, de l'avoine, de l'épeautre, du sarrasin, du maïs, du seigle, des pois, du soja et du lupin. Parmi les fibres de céréales, le son de blé est particulièrement préféré.

Selon une forme de réalisation avantageuse du procédé conforme à la présente Invention, l'étape d'incubation est réalisée à une température comprise entre 20 et 40°C environ, pendant 12 à 48 heures environ, de préférence sous agitation modérée.

Au sens de la présente Invention, on entend par agitation modérée, toute agitation comprise entre 1 et 70 tours par minute.

Selon une forme de réalisation particulièrement préférée du procédé selon l'Invention, cette étape d'incubation est réalisée à une température de 30°C environ pendant 24 heures environ.

Selon l'Invention, la quantité de bactéries lactiques homofermentaires présente dans le milieu liquide au début de l'étape de d'incubation est de préférence comprise entre 10⁶ et 10⁹ bactéries par gramme de milieu liquide total.

Au sens de la présente Invention, on entend par bactérie lactique *Lactobacillus plantarum, Lactobacillus paraplantarum* et *Lactobacillus pentosus,* toutes bactéries lactiques aptes à fermenter un mélange de fibres végétales riche en fibres insolubles et dont l'identité a été confirmée par hybridation ADN/ADN et présentant ainsi au moins 87 % d'homologie par rapport aux souches de référence *Lactobacillus plantarum* ATCC 14917, *Lactobacillus paraplantarum* DSM10667 (Deutsche Sammlung von Mikroorganismen) et *Lactobacillus pentosus* ATCC 8041 respectivement. Un tel génotypage peut notamment être réalisé à l'aide de la technique REP-PCR (*Repetitive Extragenic Palindromic-Polymerase Chain Reaction*) décrite par Gevers *et al.* (FEMS Microbiol. Lett., 2001, **205,** 31-36).

De préférence, les bactéries lactiques utilisées selon le procédé conforme à l'Invention sont choisies parmi celles présentant une cinétique d'acidification rapide du milieu à fermenter qui traduit une bonne implantation des bactéries dans ledit milieu et une utilisation rapide des sucres disponibles.

Au sens de la présente Invention, on entend par cinétique d'acidification rapide, une vitesse d'acidification maximale du milieu à fermenter supérieure à 0,45 unités pH perdues par heure ; cette vitesse maximale étant de préférence atteinte en moins de 5 heures d'incubation.

De plus, les bactéries lactiques utilisées selon le procédé conforme à l'Invention sont de préférence choisies parmi celles permettant d'atteindre un pH de 5 en moins de 6 heures d'incubation dans le milieu en fin de fermentation.

Les bactéries lactiques utilisées selon le procédé conforme à l'Invention sont de préférence choisies parmi les souches *Lactobacillus plantarum* déposée le 27 mai 2003 sous le numéro 1-3045, *Lactobacillus plantarum* déposée le 7 juillet 2003 sous le n° 1-3064, *Lactobacillus paraplantarum* déposée le 7 juillet 2003 sous le n° I-3065 et *Lactobacillus pentosus* déposées le 4 avril 2002 sous le numéro 1-2847 et le 7 juillet 2003 sous le n° 1-3066 ; ces dépôts ayant été effectués auprès de la CNCM (Collection Nationale de Cultures de Microorganismes) tenue par l'Institut Pasteur, 25 rue du Docteur Roux, à Paris.

Les souches de bactéries lactiques 1-3045, 1-3064, I-3065, I-2847 et I-3066 précitées qui sont utilisées selon le procédé conforme à l'Invention sont nouvelles en soi et constituent à ce titre un autre objet de l'Invention.

Le milieu liquide à fermenter peut en outre comprendre au moins une levure choisie parmi *Saccharomyces cerevisiae, Saccharomyces exiguus* et *Hansenula anomala,* de préférence *Saccharomyces cerevisiae.*

Lorsqu'elles sont utilisées, la quantité de levures présente au sein du milieu liquide au début de la période d'incubation est de préférence comprise entre 10⁵ et 10⁷ levures par gramme de milieu liquide à fermenter.

Selon une forme de réalisation particulière de l'Invention, le milieu liquide à fermenter peut en outre éventuellement renfermer de environ 0,05 à 2 % en poids par rapport au poids total du milieu d'un auxiliaire technique choisi parmi le sirop d'orge malté et les amylases et protéases d'origine bactérienne ou fongique.

Les Inventeurs ont en effet mis en évidence que l'ajout de levure(s) et/ou d'un auxiliaire technique tel que l'orge malté facilite l'implantation des bactéries lactiques dans le milieu à fermenter.

Selon une autre forme de réalisation particulière de l'Invention, le milieu liquide à fermenter peut en outre renfermer des minéraux et des vitamines activateurs de fermentation, qui sont de préférence apportés par du germe de céréale à hauteur de 0,05 à 2 % du milieu total.

Selon encore une autre forme de réalisation particulière de l'Invention, le milieu liquide à fermenter peut éventuellement renfermer de 2,5 à 6 % d'un ou de plusieurs additifs tel que par exemple du sucre et en particulier du sucre semoule.

A la fin de la période d'incubation, le milieu liquide fermenté obtenu est directement utilisable à titre d'ingrédient dans des produits alimentaires crus ou cuits.

Il peut également être stocké en vue d'une utilisation ultérieure. Dans ce cas, le stockage est de préférence effectué à une température comprise entre 2 et 6°C environ, et encore plus préférentiellement à une température de 4°C environ, pendant 2 à 7 jours jusqu'à son utilisation. De plus, le stockage du milieu liquide fermenté à une telle température présente l'avantage de ralentir fortement le processus de fermentation.

La présente Invention a donc également pour objet le milieu liquide fermenté susceptible d'être obtenu par la mise en oeuvre du procédé conforme à l'Invention et tel que précédemment décrit.

Selon une forme de réalisation avantageuse de la présente Invention, le pH du milieu liquide fermenté est compris entre 3,4 et 4,5 environ avec une acidité totale titrable (ATT) comprise entre 80 et 450 mEq/kg à la fin de la période d'incubation.

Par ailleurs, les Inventeurs ont constaté que le milieu liquide fermenté peut posséder une proportion de fibres solubles au moins égale au double de celle du milieu de départ non fermenté. Cette augmentation du taux de fibres solubles traduit une hydrolyse partielle des fibres insolubles en fibres solubles lors du procédé de fermentation. L'augmentation de la proportion, initialement très faible, de fibres solubles dans le milieu fermenté présente un intérêt certain dans la mesure où les fibres solubles ont des propriétés physiologiques différentes de celles des fibres insolubles, comme par exemple la prévention du diabète et du cholestérol, la stimulation sélective, dans l'intestin, de la croissance de bactéries ayant un effet bénéfique sur la santé de leur hôte (effet probiotique des fibres solubles). Par la hausse du taux de fibres solubles au cours de la fermentation, ces propriétés s'ajoutent donc à celles des fibres insolubles (effets sur le transit intestinal notamment).

Un troisième objet de la présente Invention réside dans l'utilisation d'un tel milieu liquide fermenté à titre d'ingrédient pour la fabrication de produits alimentaires crus ou cuits.

Selon une forme de réalisation avantageuse de la présente Invention, le milieu liquide fermenté est utilisé à titre d'ingrédient pour la fabrication de produits alimentaires cuits à base de farine tels que des produits de panification comme par exemple des pains moelleux, des biscuits, des crackers, des viennoiseries, des pâtisseries, etc.

Dans ce cas, le milieu liquide fermenté représente de préférence de 5 à 60 % en poids par rapport au poids total des ingrédients, et encore plus préférentiellement de 10 à 50 %.

Lorsque le milieu liquide fermenté selon l'Invention est utilisé dans la fabrication de produits alimentaires crus, ces derniers sont de préférence choisis parmi les boissons comme les jus de fruits, les fourrages, et les produits laitiers tels que les yaourts brassés ou le lait fermenté.

Dans ce cas, et de façon avantageuse, le milieu fermenté représente alors de 3 à 25 % en poids par rapport au poids total du produit alimentaire cru fini.

Dans le cas où le milieu fermenté est utilisé à titre d'ingrédient dans la fabrication de produits crus, il est préférable de n'ajouter aucune levure au milieu liquide de départ à fermenter.

La présente Invention a également pour objet un procédé de fabrication de produits alimentaires crus ou cuits comprenant au moins une étape d'incubation d'un milieu fermenté tel que défini ci-dessus.

Selon une première forme de réalisation, il s'agit d'un procédé de préparation d'un produit alimentaire cuit qui comprend avantageusement une étape d'incorporation d'au moins un milieu liquide fermenté conforme à l'Invention et tel que défini précédemment, en mélange avec les ingrédients d'une pâte à base de farine, à la place d'une partie ou de la totalité du levain-chef qui est habituellement utilisé dans les procédés conventionnels de fabrication de produits cuits.

Au sens de la présente Invention, on entend par levain-chef le premier levain qui est préparé lors d'un procédé de fabrication de produits de panification pour la première fois et qui est par la suite simplement rafraîchi et renouvelé lors des fabrications ultérieures.

Selon une deuxième forme de réalisation, il s'agit d'un procédé de fabrication de produits alimentaires crus du type boissons (jus de fruits), fourrages ou produits laitiers (yaourts brassés, lait fermenté) dans lequel le milieu fermenté est de préférence ajouté directement aux produits une fois fabriqués.

Le procédé de fabrication conforme à l'Invention présente donc l'avantage d'être applicable sur une ligne industrielle classique, sans modification de l'appareillage en dehors de l'étape de préfermentation et permet ainsi la fabrication de produits crus ou cuits après incorporation du milieu liquide fermenté.

La présente Invention a enfin pour objet des produits alimentaires crus ou cuits renfermant des fibres végétales fermentées obtenues selon le procédé de préparation tel que défini précédemment et obtenus par le procédé de fabrication utilisant le milieu liquide fermenté en tant qu'ingrédient.

Les produits alimentaires crus ou cuits ainsi obtenus renferment de 2 à 10 fois plus de fibres totales que les produits alimentaires de l'art antérieur contenant des fibres non fermentées ou fermentées par des bactéries lactiques différentes de *Lactobacillus plantarum, Lactobacillus paraplantarum* et *Lactobacillus pentosus.*

Outre les dispositions qui précèdent, l'Invention comprend encore d'autres dispositions qui ressortiront de la description qui va suivre, qui se réfère à des exemples de préparation de milieux fermentés conformément à l'Invention, à un exemple de préparation de produits alimentaires cuits de type "cracker" ; "Petit brun Extra®" ; pain moelleux de type Captain® et pain au lait, à l'aide de milieux fermentés conformément à l'Invention, ainsi qu'à un exemple de préparation de milieux fermentés et à leurs utilisations pour la fabrication d'un jus de fruits ou d'un produit laitier.

Il doit être bien entendu toutefois que ces exemples sont donnés uniquement à titre d'illustration de l'objet de l'Invention dont ils ne constituent en aucune manière une limitation.

### EXEMPLE 1 : PREPARATION D'UN MILIEU FERMENTE (MF1)

On prépare un milieu contenant les ingrédients suivants :
- 75 % d'eau à 35°C,
- 0,02 % de bactérie *Lactobacillus pentosus* 1-2847,
- 0,01 % de levure pressée (*Saccharomyces cerevisiae)* vendue sous la référence S47 par la société Lesaffre,
- 23 % de son de blé,
- 1,2 % de germe de blé,
- 1,2 % de sirop d'orge malté vendu sous la dénomination Diamalt par la société Brewing Products.

Le milieu ainsi préparé comprend environ 9.10⁵ levures et 2,3.10⁶ bactéries par gramme de milieu total avant fermentation. Après une incubation à 35°C pendant 19 heures sous agitation à 2 tours par minute, le milieu contient environ 9,5.10⁶ levures et 2,6.10⁸ bactéries par gramme de milieu fermenté total et possède un pH de 4,5 en fin de fermentation et une ATT de 220 mEq/kg. Le milieu fermenté ainsi obtenu est ensuite conservé en cuve à 4°C jusqu'à son utilisation.

### EXEMPLE 2 : PREPARATION D'UN MILIEU FERMENTE (MF2)

On prépare un milieu contenant les ingrédients suivants :
- 56 % d'eau à 40°C,
- 0,2 % de bactérie *Lactobacillus plantarum* 1-3045,
- 42 % de son de blé,
- 1 % de germe de blé,
- 1 % de sirop d'orge malté vendu sous la dénomination Diamalt par la société Brewing products.

Le milieu ainsi préparé comprend environ 10⁷ bactéries par gramme de milieu total avant fermentation. Après une incubation à 35°C pendant 24 heures sous agitation à 2 tours par minute, le milieu contient environ 10⁸ bactéries par gramme de milieu fermenté total, possède un pH de 3,8 en fin de fermentation et une ATT de 400 mEq/kg. Le milieu fermenté ainsi obtenu peut être utilisé directement ou stocké à 4°C jusqu'à son utilisation.

### EXEMPLE 3 : PREPARATION D'UN MILIEU FERMENTE (MF3)

On prépare un milieu contenant les ingrédients suivants :
- 58 % d'eau,
- 0,01 % de bactérie *Lactobacillus plantarum* I-3045,
- 0,04 % de levure pressée S47,
- 42 % de son de blé,
- 4 % de sucre semoule.

Le milieu ainsi préparé comprend environ 2,5.10⁶ levures et 10⁷ bactéries par gramme de milieu total avant fermentation. Après une incubation à 28°C pendant 24 heures sans agitation, le milieu contient environ 10⁷ levures et 2.10⁷ bactéries par gramme de milieu fermenté total, possède un pH de 4 en fin de fermentation et une ATT de 330 mEq/kg. Le milieu fermenté ainsi obtenu peut être utilisé directement ou stocké à 4°C jusqu'à son utilisation.

### EXEMPLE 4 : PREPARATION D'UN MILIEU FERMENTE (MF4)

On prépare un milieu contenant les ingrédients suivants :
- 56 % d'eau,
- 0,01 % de bactérie *Lactobacillus plantarum* 1-3045
- 0,04 % de levure pressée S47,
- 40 % de son de blé,
- 4 % de sucre semoule.

Le milieu ainsi préparé comprend environ 9.10⁵ levures et 2,3.10⁶ bactéries par gramme de milieu total avant fermentation. Après une incubation à 28°C pendant 24 heures sans agitation, le milieu contient environ 5.10⁶ levures et 1.10⁷ bactéries par gramme de milieu fermenté total, possède un pH de 4,2 en fin de fermentation et une ATT de 300 mEq/kg. Le milieu fermenté ainsi obtenu peut être utilisé directement ou stocké à 4°C jusqu'à son utilisation.

### EXEMPLE 5 : PREPARATION D'UN PRODUIT ALIMENTAIRE CUIT DE TYPE CRACKER

Une pâte est réalisée par mélange des ingrédients suivants et dans l'ordre énoncé :
- 39 % de milieu fermenté MF1 obtenu par le procédé tel qu'il est décrit dans l'exemple 1,
- 36 % de farine de blé tendre type 65,
- 9 % d'huile de palme,
- 12 % de son de blé,
- 1 % de sel,
- 1 % de bicarbonate de soude (BCS),
- 2 % d'eau.

Le mélange ainsi obtenu est pétri dans un pétrin vertical pendant 12 minutes à une vitesse de 50 tours par minute, et étuvé pendant 1 heure à 26°C dans une atmosphère présentant une humidité relative de 80 %.

Le feuilletage de la pâte s'effectue en couches empilées, le laminage est progressif jusqu'à une épaisseur de préférence égale à 1 mm. La cuisson des pâtons découpés par l'emporte-pièce Saïwa Prémium® est de 7 minutes à 260°C.

Les produits finis obtenus possèdent ainsi une teneur en fibres totales égale à 22,55 %, comparativement à des crackers du type Saïwa Prémium® préparés par un procédé de préparation conventionnel et qui possèdent seulement 3,8 % de fibres totales.

### EXEMPLE 6 : PREPARATION D'UN BISCUIT DE TYPE PETIT BRUN EXTRA®

Une pâte est réalisée par mélange des ingrédients suivants :
- 20 % de milieu fermenté MF2 obtenu par le procédé tel qu'il est décrit dans l'exemple 2,
- 42 % de farine de blé tendre type 55,
- 9 % d'huile de palme,
- 0,4 % de BCS,
- 7 % de sucre glace,
- 4 % de sucre inverti,
- 16 % de sirop d'orge malté vendu par la société Brewing Products,
- 1 % d'eau.

La pâte est réalisée dans un pétrin double bras horizontal par un mélange préalable des poudres (farine, sucre glace, BCS) avec le milieu fermenté MF2 et le sucre inverti à une vitesse de 40 tours par minute pendant 1 minute 30. L'ensemble des liquides (eau, huile de palme) est alors ajouté et le mélange est pétri à une vitesse de 40 tours par minute pendant 1 minute 30. Les pâtons découpés par l'empreinte Petit Brun Extra® sont cuits à une température de 210°C pendant 5 à 7 minutes.

Les produits finis obtenus possèdent ainsi une teneur en fibres totales égale à 5,22 %, comparativement à des biscuits Petit Brun Extra® préparés par un procédé de préparation conventionnel et qui possèdent seulement 2,6% de fibres totales.

### EXEMPLE 7 : PREPARATION D'UN PAIN MOELLEUX DE TYPE CAPTAIN®

Une pâte est réalisée par mélange des ingrédients suivants :
- 41 % de milieu fermenté MF3 obtenu par le procédé tel qu'il est décrit dans l'exemple 3,
- 13 % de farine de blé tendre type 45,
- 0,5 % de BCS,
- 0,3 % de sel,
- 18 % d'oeufs entiers,
- 16 % de sucre semoule,
- 4 % de glycérol,
- 2 % de lait entier,
- 6 % d'huile de colza.

La pâte est réalisée dans un pétrin planétaire par un mélange préalable des ingrédients en poudre (farine, BCS, sel, sucre semoule) avec le milieu MF3 à une vitesse de 40 tours par minute pendant 1 minute. Tous les ingrédients liquides (glycérol, lait entier, oeufs, huile de colza) sont ensuite ajoutés excepté l'huile de colza et le mélange est pétri à une vitesse de 40 tours par minute pendant 1 minute puis à une vitesse de 70 tours par minute pendant 1 minute supplémentaire. Enfin, l'huile de colza est ajoutée au mélange ainsi obtenu qui est alors pétri à nouveau à une vitesse de 40 tours par minute pendant 1 minute.

Après un repos de 30 minutes en bol, la pâte est répartie dans un moule compartimenté en petits caissons rectangulaires à raison de 27 grammes par caisson. Enfin les produits moulés sont cuits à une température de 210°C pendant 12 à 16 minutes, et de préférence pendant 13 minutes.

Les produits finis obtenus possèdent ainsi une teneur en fibres totales égale à 9,03 %, comparativement à des pains moelleux de type Captain® préparés par un procédé de préparation conventionnel et qui possèdent seulement 2,8% de fibres totales.

### EXEMPLE 8 : PREPARATION D'UN PRODUIT DE VIENNOISERIE DE TYPE PAIN AU LAIT

Une pâte est réalisée par mélange des ingrédients suivants :
- 47 % de milieu fermenté MF4 obtenu par le procédé tel qu'il est décrit dans l'exemple 4,
- 38 % de farine de blé tendre type 45,
- 4 % de sucre semoule,
- 0,7 % d'oeufs en poudre,
- 0,8 % de glycérine,
- 4 % d'huile de colza,
- 6 % de levures osmophiles,
- 0 03 % d'une solution à 10 % d'acide ascorbique,
- 0,12 % de sel fin

La pâte est réalisée dans un pétrin Artofex® par un mélange préalable du gluten, de la levure et du milieu MF4 à une vitesse de 40 tours par minute pendant 1 minute. Le reste des ingrédients est ensuite ajouté et le mélange est pétri à une vitesse de 70 tours par minute pendant 15 minutes supplémentaires.

Le boulage de la pâte est effectué à raison de 28 grammes par pâton et les pâtons sont ensuite mis à étuver pendant 50 minutes à 38°C dans une atmosphère présentant une humidité relative de 80 %. Enfin les produits moulés sont cuits à une température de 200°C pendant 12 à 16 minutes, et de préférence pendant 14 minutes.

Les produits alimentaires finis obtenus possèdent ainsi une teneur en fibres totales égale à 11,07 %, comparativement à des pains au lait préparés par un procédé de préparation conventionnel et qui possèdent seulement 3,9 % de fibres totales.

### EXEMPLE 9 : PREPARATION D'UN MILIEU FERMENTE (MF5) ET UTILISATION D'UN TEL MILIEU POUR LA FABRICATION D'UN JUS DE FRUITS

On prépare un milieu contenant les ingrédients suivants :
- 84 % d'eau,
- 0,01 % de bactérie *Lactobacillus plantarum* 1 3045,
- 3 % de sucre inverti,
- 2,99 % de son de blé.

Après une incubation à 28°C pendant 24 heures et sous agitation à 30 tours par minute, le milieu contient environ 1.10⁹ bactéries par gramme de milieu fermenté total, possède un pH de 3,9 en fin de fermentation et une ATT de 120 mEq/kg.

Le milieu ainsi fermenté est ajouté à raison de 10 à 20 % dans un jus de fruits, par exemple de pêche ou de poire.

### EXEMPLE 10 : PREPARATION D'UN MILIEU FERMENTE (MF6) ET UTILISATION D'UN TEL MILIEU POUR LA FABRICATION D'UN PRODUIT LAITIER

On prépare un milieu contenant les ingrédients suivants :
- 90 % d'eau,
- 0,01 % de bactérie *Lactobacillus plantarum* I 3045,
- 1,99 % de sucre inverti,
- 8 % de son de blé.

Après une incubation à 28°C pendant 24 heures et sous agitation à 30 tours par minute, le milieu contient environ 1.10⁹ bactéries par gramme de milieu fermenté total, possède un pH de 3,9 en fin de fermentation et une ATT de 170 mEq/kg.

Le milieu ainsi fermenté est ajouté à raison de 3 à 10 % dans un lait fermenté ou un yaourt brassé.

L'ensemble de ces résultats démontrent clairement que le milieu fermenté préparé selon le procédé conforme à l'Invention et utilisé en tant qu'ingrédient dans la fabrication de produits alimentaires crus ou cuits permet d'incorporer des quantités importantes de fibres dans les produits finis, et ce en augmentant d'un facteur allant de 2 à 10 le taux d'incorporation de fibres totales dans les produits alimentaires ainsi obtenus.

## Revendications

1. Procédé de préparation d'un milieu liquide fermenté à base de fibres végétales solubles et insolubles, **caractérisé par le fait qu'**il comprend au moins une étape d'incubation d'un milieu liquide comprenant de l'eau et au moins un mélange de fibres végétales, avec au moins une bactérie lactique homofermentaire choisie parmi les *Lactobacillus plantarum, Lactobacillus paraplantarum* et *Lactobacillus pentosus ;* ledit mélange de fibres végétales renfermant au moins 40 % en poids de fibres insolubles par rapport au poids total de fibres ; le substrat végétal contenant les fibres n'étant ni préhydrolysé ou prédigéré enzymatiquement ni gélatinisé et sa teneur en fibres totales étant d'au moins 20 % en poids par rapport au poids total de sa matière sèche.

2. Procédé selon la revendication 1, **caractérisé en ce que** le milieu liquide comprend de 55 à 90 % d'eau et 10 à 45 % de fibres végétales.

3. Procédé selon l'une quelconque des revendications précédentes, **caractérisé en ce que** le mélange de fibres végétales est un mélange de fibres de céréales et/ou de légumineuses.

4. Procédé selon la revendication 3, **caractérisé en ce que** les fibres de céréales et de légumineuses sont choisies parmi les fibres extraites du blé, de l'orge, de l'avoine, de l'épeautre, du sarrasin, du maïs, du seigle, des pois, du soja et du lupin.

5. Procédé selon la revendication 4, **caractérisé en ce que** les fibres de céréales sont du son de blé.

6. Procédé selon l'une quelconque des revendications précédentes, **caractérisé en ce que** l'étape d'incubation est réalisée à une température comprise entre 20 et 40°C pendant 12 à 48 heures.

7. Procédé selon l'une quelconque des revendications précédentes, **caractérisé en ce que** l'étape d'incubation est réalisée sous agitation modérée.

8. Procédé selon l'une quelconque des revendications précédentes, **caractérisé en ce que** la quantité de bactéries lactiques homofermentaires présente dans le milieu liquide au début de l'étape d'incubation est comprise entre 10⁶ et 10⁹ bactéries par gramme de milieu total.

9. Procédé selon l'une quelconque des revendications précédentes, **caractérisé en ce que** les bactéries lactiques utilisées sont choisies parmi celles présentant une vitesse d'acidification maximale du milieu à fermenter supérieure à 0,45 unités pH perdues par heure.

10. Procédé selon l'une quelconque des revendications précédentes, **caractérisé en ce que** les bactéries lactiques utilisées sont choisies parmi les souches *Lactobacillus plantarum* I-3045 et I-3064, *Lactobacillus paraplantarum* I-3065 et *Lactobacillus pentosus* I-2847 et I-3066.

11. Procédé selon l'une quelconque des revendications précédentes, **caractérisé en ce que** le milieu comprend en outre au moins une levure choisie parmi *Saccharomyces cerevisiae, Saccharomyces exiguus* et *Hansenula anomala.*

12. Procédé selon l'une quelconque des revendications précédentes, **caractérisé en ce que** la quantité de levures présente au sein du milieu liquide au début de la période d'incubation est comprise entre 10⁵ et 10⁷ levures par gramme de milieu liquide à fermenter.

13. Procédé selon l'une quelconque des revendications précédentes, **caractérisé en ce que** le milieu comprend en outre de 0,05 à 2 % d'un auxiliaire technique choisi parmi le sirop d'orge malté et les amylases et protéases d'origine bactérienne ou fongique.

14. Procédé selon l'une quelconque des revendications précédentes, **caractérisé en ce que** le milieu comprend en outre des minéraux et des vitamines activateurs de fermentation.

15. Procédé selon la revendication 14, **caractérisé en ce que** les minéraux et les vitamines activateurs de fermentation sont apportés par du germe de céréale à hauteur de 0,05 à 2 % du milieu total.

16. Procédé selon l'une quelconque des revendications précédentes, **caractérisé en ce que** le milieu comprend en outre de 2,5 à 6 % de sucre.

17. Procédé de fabrication de produits alimentaires crus ou cuits, **caractérisé en ce qu'**il comprend la préparation d'un milieu liquide fermenté à base de fibres végétales solubles et insolubles comprenant au moins une étape d'incubation selon le procédé défini à l'une quelconque des revendications 1 à 16.

18. Procédé de fabrication de produits alimentaires cuits selon la revendication 17, **caractérisé en ce qu'**il comprend l'incorporation dudit milieu liquide fermenté en mélange avec les ingrédients d'une pâte à base de farine, à la place d'une partie ou de la totalité du levain-chef.

## Claims

1. Process for the preparation of a fermented liquid medium based on soluble and insoluble vegetable fibres, **characterized in that** it comprises at least one incubation step of a liquid medium comprising water and at least one mixture of vegetable fibres with at least one homofermentary lactic acid bacterium selected from *Lactobacillus plantarum, Lactobacillus paraplantarum* and *Lactobacillus pentosus,* said mixture of vegetable fibres containing at least 40% by weight of insoluble fibres, based on the total weight of fibres; the vegetable substrate containing the fibres being neither prehydrolyzed or predigested enzymatically, nor gelatinized, and its total fibre content being at least 20% by weight, based on the total weight of its dry matter.

2. Process according to Claim 1, **characterized in that** the liquid medium comprises from 55 to 90% of water and 10 to 45% of vegetable fibres.

3. Process according to either one of the preceding claims, **characterized in that** the mixture of vegetable fibres is a mixture of cereal and/or leguminous fibres.

4. Process according to Claim 3, **characterized in that** the cereal and leguminous fibres are selected from fibres extracted from wheat, barley, oats, spelt, buckwheat, maize, rye, peas, soya and lupin.

5. Process according to Claim 4, **characterized in that** the cereal fibres are wheat bran.

6. Process according to any one of the preceding claims, **characterized in that** the incubation step is carried out at a temperature between 20 and 40°C for 12 to 48 hours.

7. Process according to any one of the preceding claims, **characterized in that** the incubation step is carried out with moderate agitation.

8. Process according to any one of the preceding claims, **characterized in that** the amount of homofermentary lactic acid bacteria present in the liquid medium at the start of the incubation step is between 10⁶ and 10⁹ bacteria per gram of total medium.

9. Process according to any one of the preceding claims, **characterized in that** the lactic acid bacteria used are selected from those for which the maximum acidification rate of the fermentation medium is greater than 0.45 pH unit lost per hour.

10. Process according to any one of the preceding claims, **characterized in that** the lactic acid bacteria used are selected from the strains *Lactobacillus plantarum* 1-3045 and 1-3064, *Lactobacillus paraplantarum* I-3065 and *Lactobacillus pentosus* 1-2847 and I-3066.

11. Process according to any one of the preceding claims, **characterized in that** the medium also comprises at least one yeast selected from *Saccharomyces cerevisiae, Saccharomyces exiguus* and *Hansenula anomala.*

12. Process according to any one of the preceding claims, **characterized in that** the amount of yeasts present in the liquid medium at the start of the incubation period is between 10⁵ and 10⁷ yeasts per gram of liquid fermentation medium.

13. Process according to any one of the preceding claims, **characterized in that** the medium also comprises from 0.05 to 2% of a technical auxiliary selected from malted barley syrup and amylases and proteases of bacterial or fungal origin.

14. Process according to any one of the preceding claims, **characterized in that** the medium also comprises minerals and vitamins that activate fermentation.

15. Process according to Claim 14, **characterized in that** the minerals and vitamins that activate fermentation are introduced via cereal germ in an amount of 0.05 to 2% of the total medium.

16. Process according to any one of the preceding claims, **characterized in that** the medium also comprises from 2.5 to 6% of sugar.

17. Process for the manufacture of raw or cooked food products, **characterized in that** it comprises the preparation of a fermented liquid medium based on soluble and insoluble vegetable fibres, comprising at least one incubation step according to the process defined in any one of Claims 1 to 16.

18. Process for the manufacture of cooked food products according to Claim 17, **characterized in that** it comprises the incorporation of said fermented liquid medium in a mixture with the ingredients of a flour-based dough, in place of all or part of the levain chef.

## Patentansprüche

1. Verfahren zur Herstellung eines fermentierten flüssigen Mediums auf der Basis von löslichen und unlöslichen pflanzlichen Fasern, **dadurch gekennzeichnet, dass** es wenigstens eine Inkubationsstufe eines flüssigen Mediums, das Wasser und wenigstens ein Gemisch pflanzlicher Fasern umfasst, mit wenigstens einem homofermentativen Milchsäurebakterium, ausgewählt aus *Lactobacillus plantarum, Lactobacillus paraplantarum* und *Lactobacillus pentosus,* umfasst, wobei das Gemisch pflanzlicher Fasern wenigstens 40 Gew.-% unlösliche Fasern, bezogen auf das Gesamtgewicht der Fasern, umfasst; wobei das Pflanzensubstrat, das die Fasern enthält, weder vorhydrolisiert oder enzymatisch vorverdaut noch gelatiniert wurde und sein Gehalt an Gesamtfasern wenigstens 20 Gew.-%, bezogen auf das Gesamtgewicht seiner Trockensubstanz, ist.

2. Verfahren nach Anspruch 1, **dadurch gekennzeichnet, dass** das flüssige Medium 55 bis 90% Wasser und 10 bis 45% pflanzliche Fasern umfasst.

3. Verfahren nach einem der vorangehenden Ansprüche, **dadurch gekennzeichnet, dass** das Gemisch pflanzlicher Fasern ein Gemisch aus Fasern von Getreiden und/oder von Leguminosen ist.

4. Verfahren nach Anspruch 3, **dadurch gekennzeichnet, dass** die Fasern von Getreiden und Leguminosen ausgewählt sind aus Fasern, die aus Weizen, Gerste, Hafer, Dinkel, Buchweizen, Mais, Roggen, Erbsen, Soja und Lupine gewonnen werden.

5. Verfahren nach Anspruch 4, **dadurch gekennzeichnet, dass** die Fasern von Getreide aus Weizenkleie sind.

6. Verfahren nach einem der vorangehenden Ansprüche, **dadurch gekennzeichnet, dass** die Inkubationsstufe bei einer Temperatur zwischen 20 und 40°C während 12 bis 48 Stunden durchgeführt wird.

7. Verfahren nach einem der vorangehenden Ansprüche, **dadurch gekennzeichnet, dass** die Inkubation unter mäßigem Rühren durchgeführt wird.

8. Verfahren nach einem der vorangehenden Ansprüche, **dadurch gekennzeichnet, dass** die Menge an homofermentativen Milchsäurebakterien, die zu Beginn der Inkubationsstufe in dem flüssigen Medium vorliegt, zwischen 10⁶ und 10⁹ Bakterien pro Gramm Gesamtmedium liegt.

9. Verfahren nach einem der vorangehenden Ansprüche, **dadurch gekennzeichnet, dass** die verwendeten Milchsäurebakterien unter denen ausgewählt werden, die eine maximale Säuerungsgeschwindigkeit des zu fermentierenden Mediums über 0,45 pH-Einheiten, die pro Stunde verloren gehen, aufweisen.

10. Verfahren nach einem der vorangehenden Ansprüche, **dadurch gekennzeichnet, dass** die verwendeten Milchsäurebakterien unter den Stämmen *Lactobacillus plantarum* 1-3045 und 1-3064, *Lactobacillus paraplantarum* 1-3065 und *Lactobacillus pentosus* 1-2847 und 1-3066 ausgewählt werden.

11. Verfahren nach einem der vorangehenden Ansprüche, **dadurch gekennzeichnet, dass** das Medium außerdem wenigstens eine Hefe umfasst, die aus *Saccharomyces cerevisiae, Saccharomyces exiguus* und *Hansenula anomala* ausgewählt wird.

12. Verfahren nach einem der vorangehenden Ansprüche, **dadurch gekennzeichnet, dass** die Menge an Hefen, die im flüssigen Medium zu Beginn des Inkubationszeitraums vorliegt, zwischen 10⁵ und 10⁷ Hefen pro Gramm zu fermentierendes flüssiges Medium ist.

13. Verfahren nach einem der vorangehenden Ansprüche, **dadurch gekennzeichnet, dass** das Medium außerdem 0,05 bis 2% eines technischen Hilfsmittels, ausgewählt unter Gerstenmalzsirup und den Amylasen und Proteasen bakterieller Herkunft oder fungaler Herkunft, umfasst.

14. Verfahren nach einem der vorangehenden Ansprüche, **dadurch gekennzeichnet, dass** das Medium außerdem Mineralstoffe und Vitamine als Fermentationsaktivatoren umfasst.

15. Verfahren nach Anspruch 14, **dadurch gekennzeichnet, dass** die Mineralstoffe und die Vitamine als Fermentationsaktivatoren durch Getreidekeime in einer Menge von 0,05 bis 2% des gesamten Mediums eingebracht werden.

16. Verfahren nach einem der vorangehenden Ansprüche, **dadurch gekennzeichnet, dass** das Medium außerdem 2,5 bis 6% Zucker umfasst.

17. Verfahren zur Herstellung von rohen oder gegarten Lebensmittelprodukten, **dadurch gekennzeichnet, dass** es die Herstellung eines fermentierten flüssigen Mediums auf der Basis von löslichen und unlöslichen pflanzlichen Fasern umfasst, das wenigstens eine Inkubationsstufe nach dem Verfahren, das in einem der Ansprüche 1 bis 16 definiert ist, umfasst.

18. Verfahren zur Herstellung von gegarten bzw. gekochten Lebensmittelprodukten nach Anspruch 17, **dadurch gekennzeichnet, dass** es die Einarbeitung des genannten fermentierten flüssigen Mediums im Gemisch mit den Ingredienzien eines Teigs auf Mehlbasis anstelle eines Teils oder der Gesamtheit der Sauerteiggrundmasse (levain-chef) umfasst.
